# EUROPEAN PATENT APPLICATION

(11) **EP 1 878 729 A1**
(43) Date of publication of application: **16.01.2008**
(21) Application number: 07113176.7
(22) Date of filing: 22.09.2003
(51) Int. Cl.: C07D 257/04

(54) **INTERMEDIATE FOR THE MANUFACTURE OF VALSARTAN**

(30) Priority: 23.09.2002 GB 0222056
(62) Divisional of application: 03750599.7
(71) Applicant: Novartis AG, 4002 Basel (CH); Novartis Pharma GmbH, 1230 Vienna (AT)
(72) Inventor: Hirt, Hans, 4153, Reinach BL (CH); Sedelmeier, Gottfried, 79227, Schallstadt (DE); Krell, Christoph, 4057, Basel (CH)
(74) Representative: Hillebrand, Dirk

(57) **Abstract**

The present invention relates to a process for the manufacture of an angiotensin receptor blocker (ARB; also called angiotension II receptor antagonist or AT₁ receptor antagonist) and salts thereof, to novel intermediates and process steps.

## Description

The present invention relates to a process for the manufacture of an angiotensin receptor blocker (ARB; also called angiotension II receptor antagonist or A T₁ receptor antagonist) and salts thereof, to novel intermediates and process steps. ARBs can, for example, be used for the treatment of hypertension and related diseases and conditions.

The class of ARBs comprises compounds having different structural features, essentially preferred are the non-peptidic ones. For example, mention may be made of the compounds that are selected from the group consisting of valsartan (cf. EP 443983), losartan (cf. EP253310), candesartan (cf. 459136), eprosartan (cf. EP 403159), irbesartan (cf. EP454511), olmesartan (cf. EP 503785), and tasosartan (cf. EP539086), or, in each case, a pharmaceutically acceptable salt thereof.

All of these ARBs comprise the following common structural element:

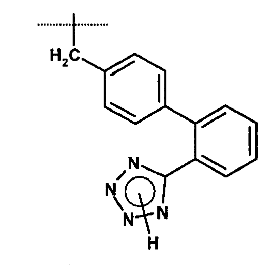

The formation of the tetrazole ring is a critical step in the manufacture of these compounds. Methods for the manufacture of ARBs having this structural feature include the formation of said tetrazole ring by starting from corresponding cyano derivatives that react with HN₃ or a suitable alkali metal salt thereof such as sodium azide or with an organotin azide such as tributyltin azide or with a silyl azide. The use of azides for forming the tetrazol ring system requires a sophisticated system to run the reaction in a safe way during an up-scaled production process. Accordingly, an objective is to develop alternative process variants that exclude the use of azides in the last steps of the production of corresponding ARBs.

The objective of the present invention is to provide a synthesis of compounds of formula (I) that (1) does not require a process step where an azide is used, (2) results in high yields, (3) minimises pollution of the environment e.g. by essentially avoiding organotin compounds, (4) is economically attractive by using less reaction steps in the reaction sequence for the manufacture of compounds of formula (I), (5) affords enantiomerically pure target products and products of high crystallisability. In addition, as the tetrazole ring system is formed at an earlier stage of the reaction sequence, (6) the risk of contamination of the final product (and late intermediates) with trace amounts of tin components is much lower. Normally, the tetrazole ring is formed by reacting a corresponding cyano derivative with an organotin compound such as tributyl tin azide. For ecological reasons, the heavy metal tin and especially organotin compounds are to be handled with special care. Furthermore, (7) another objective of the present invention is to provide a process that can be carried out on a larger scale and can thus be used for a corresponding production process and to avoid e.g. racemisation and thus separation of any enantiomers.

It has surprisingly been found that the process according to the present invention meets the above objectives.

The present invention relates to a process for the manufacture of the compound of formula (I) 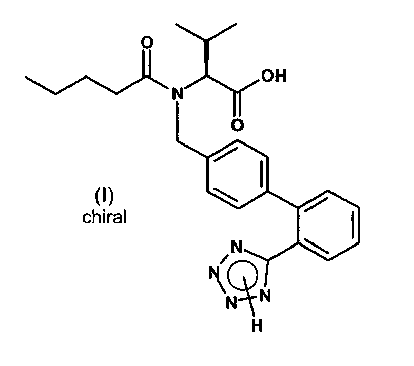 or a salt thereof, comprising
(a) reacting a compound of formula (II a) 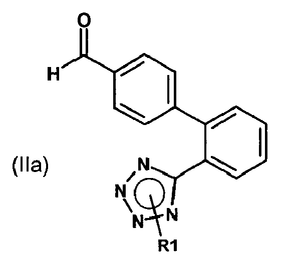 or a salt thereof, wherein R₁ is hydrogen or a tetrazole protecting group, with a compound of formula 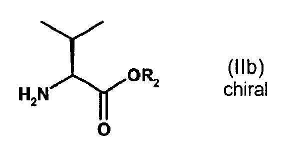 or a salt thereof, wherein R₂ represents hydrogen or a carboxy protecting group, under the conditions of a reductive amination; and
(b) acylating a resulting compound of formula (II c) 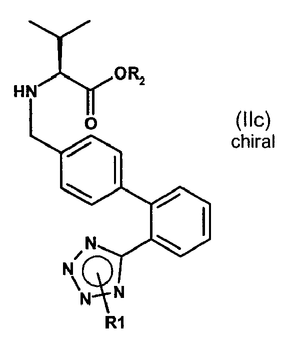 or a salt thereof with a compound of formula (II d) 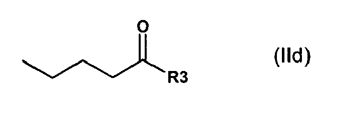 wherein R₃ is an activating group; and,
(c) if R₁ and/or R₂ are different form hydrogen, removing the protecting group(s) in a resulting compound of formula (II e) 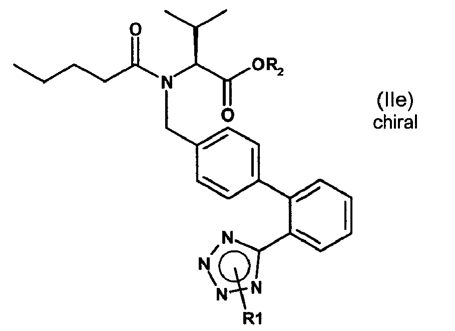 or a salt thereof; and
(d) isolating a resulting compound of formula (I) or a salt thereof; and, if desired, converting a resulting free acid of formula (I) into a salt thereof or converting a resulting salt of a compound of formula (I) into the free acid of formula (I) or converting a resulting salt of a compound of formula (I) into a different salt.

The reactions described above and below in the variants are carried out, for example, in the absence or, customarily, in the presence of a suitable solvent or diluent or a mixture thereof, the reaction, as required, being carried out with cooling, at room temperature or with warming, for example in a temperature range from about -80°C up to the boiling point of the reaction medium, preferably from about -10°C to about +200°C, and, if necessary, in a closed vessel, under pressure, in an inert gas atmosphere and/or under anhydrous conditions.

Compounds of formulae (II a), (II b), (II c) and (II e), wherein either or both of R₁ and R₂ are hydrogen can form salts with bases, as both the unprotected tetrazole ring and the unprotected carboxy group have acidic properties, while compounds of formulae (II b) and (llc) can also form salts with acids.

A corresponding tetrazole protection group (R₁) is selected from those known in the art. Especially, R₁ is selected from the group consisting of tert-C₄-C₇-alkyl such as tert-butyl; C₁-C₂-alkyl that is mono-, di or trisubstituted by phenyl, such as benzyl or benzhydryl or trityl, wherein the phenyl ring is un-substituted or substituted by one or more, e.g. two or three, residues e.g. those selected from the group consisting of tert-C₁-C₇-alkyl, hydroxy, C₁-C₇alkoxy, C₂-C₈-alkanoyl-oxy, halogen, nitro, cyano, and trifluoromethyl (CF₃); picolinyl; piperonyl; cumyl; allyl; cinnamoyl; fluorenyl; silyl such as tri-C₁-C₄-alkyl-silyl, for example, trimethyl-silyl, triethylsilyl or tert-butyl-dimethyl-silyl, or di-C₁-C₄-alkyl-phenyl-silyl, for example, dimethyl-phenyl-silyl; C₁-C₇-alkyl-sulphonyl; arylsulphonyl such as phenylsulphonyl wherein the phenyl ring is un-substituted or substituted by one or more, e.g. two or three, residues e.g. those selected from the group consisting of C₁-C₇-alkyl, hydroxy, C₁-C₇-alkoxy, C₂-C₈-alkanoyl-oxy, halogen, nitro, cyano, and CF₃; C₂-C₈-alkanoyl such as acetyl or valeroyl; and esterified carboxy such as C₁-C₇-alkoxy-carbony, for example, methoxy-, ethoxy- or tert-butyloxy-carbonyl; and allyloxycarbonyl. Examples of preferred protective groups which may be mentioned are tert-butyl, benzyl, p-methoxybenzyl, 2-phenyl-2-propyl, diphenylmethyl, di(p-methoxyphenyl)methyl, trityl, (p-methoxyphenyl)diphenylmethyl, diphenyl(4-pyridyl)methyl, benzyloxymethyl, methoxymethyl, ethoxymethyl, methylthiomethyl, 2-tetrahydropyranyl, allyl, trimethylsilyl and triethylsilyl.

A corresponding carboxy protecting group (R₂) is selected from those known in the art. Especially, R₂ is selected from the group consisting of C₁-C₇-alkyl such as methyl, ethyl or a tert-C₄-C₇-alkyl, especially tert-butyl; C₁-C₂-alkyl that is mono-, di or trisubstituted by phenyl, such as benzyl or benzhydryl, wherein the phenyl ring is un-substituted or substituted by one or more, e.g. two or three, residues e.g. those selected from the group consisting of C₁-C₇-alkyl, hydroxy, C₁-C₇-alkoxy, C₂-C₈-alkanoyl-oxy, halogen, nitro, cyano, and CF₃; picolinyl; piperonyl; allyl; cinnamyl; tetrahydrofuranyl; tetrahydropyranyl; methoxyethoxy-methyl, and benzyloxymethyl. A preferred example of protective groups which may be mentioned is benzyl.

The activating group R₃ is, for example, an activating group that is being used in the field of peptides, such as halogen such as chlorine, fluorine or bromine; C₁-C₇-alkylthio such as methyl-thio, ethyl-thio or tert-butyl-thio; pyridyl-thio such as 2-pyridyl-thio; imidazolyl such as 1-imidazolyl; benzthiazolyl-oxy such as benzthiazolyl-2-oxy-; benzotriazol-oxy such as benzotriazolyl-1-oxy-; C₂-C₈-alkanoyloxy, such as butyroyloxy or pivaloyloxy; or 2,5-dioxo-pyrrolidinyl-1-oxy.

The general terms used hereinbefore and hereinafter have the following meanings, unless defined otherwise:
C₁-C₇-Alkyl is for example methyl, ethyl, n-propyl, isopropyl, n-butyl, isobutyl, sec-butyl, tert-butyl or a corresponding pentyl, hexyl or heptyl residue. C₁-C₄-alkyl, especially methyl, ethyl or tert-butyl is preferred.
C₁-C₇-Alkoxy is for example methoxy, ethoxy, n-propyloxy, isopropyloxy, n-butyloxy, isobutyloxy, sec-butyloxy, tert-butyloxy or a corresponding pentyloxy, hexyloxy, or heptyloxy residue. C₁-C₄-alkoxy is preferred. Especially preferred is methoxy, ethoxy and tert-butoxy.
C₂-C₈-Alkanoyl in C₂-C₈alkanoyl-oxy is in particular acetyl, propionyl, butyryl, isobutyryl or pivaloyl. C₂-C₅Alkanoyl is preferred. Especially preferred is acetyl or pivaloyl.
Halogen is in particular chlorine, fluorine or bromine, and in a broader sense includes iodine. Chlorine is preferred.

### Step (a):

In reaction Step (a), the reductive amination is carried out in the presence of a reducing agent. A suitable reducing agent is a borohydride, which may also be in a complexed form, or hydrogen or a hydrogen donor both in the presence of a hydrogenation catalyst. Furthermore, a reducing agent is a suitable selenide or a silane.

A suitable borohydride or a complexed borohydride is, for example, an alkali metal borohydride such as sodium borohydride or lithium borohydride; an earth alkali metal borohydride such as calcium borohydride; an alkali metal cyanoborohydride, such as sodium cyanoborohydride or lithium cyanoborohydride, an alkali metal tri-(C₁-C₇-alkoxy)-borohydride such as sodium trimethoxy-ethoxy-borohydride; a tetra-C₁-C₇-alkylammonium-(cyano)borohydride such as tetrabutylammonium-borohydride or tetrabutylammonium-cyanoborohydride.

A suitable catalyst for the reductive amination with hydrogen or a hydrogen donor is, for example, nickel, such as Raney nickel, and noble metals or their derivatives, for example oxides, such as palladium, platinium or platinum oxide, which may be applied, if desired, to support materials, for example to carbon or calcium carbonate, for example, platinium on carbon. The hydrogenation with hydrogen or a hydrogen donor may preferably be carried out at pressures between 1 and about 100 atmosphere and at room temperature between about -80° to about 200°C, in particular between room temperature and about 100°C.

A preferred hydrogen donor is, for example, a system comprising 2-propanol and, if desired, a base, or, most preferably, formic acid or a salt thereof, e.g. an alkali metal, or tri-C₁-C₇-alkyl-ammonium salt thereof, for example, the sodium or the potassium salt thereof, if desired, in the presence of a tertiary amine, such as triethylamine. Further hydrogen donors comprise other alcohols such as ethanol, 2-methoxyethanol, benzyl alcohol, benzhydrol, pentan-2-ol, 1,2-ethandiol, 2,3-butandiol or cyclohexandiol, hydrazine, cyclohexene, cyclohexadiene, indane, tetralin, indoline, tetrahydroquinoline, hydroquinone, hypophosphinic acid or a suitable salt thereof such as the sodium salt thereof, sodium tetrahydroborate, sugars, ascorbic acid, limonene, or silanes. The hydrogen donor may also be used as solvent, especially 2-propanol or formic acid.

A suitable selenide is, for example, selenophenol which is unsubstituted or substituted. Suitable substituents comprise, for example, one, two or three substituents selected from e.g. halo, trifluoromethyl, trifluoromethoxy, C₁-C₇-alkyl, C₁-C₇-alkoxy, nitro, cyano, hydroxyl, C₂-C₁₂-alkanoyl, C₁-C₁₂-alkanoyloxy, and carboxy. Those silanes are preferred that are completely soluble in the reaction medium and that may moreover produce organic soluble by-products. Especially preferred are tri-C₁-C₇-alkyl-silanes, especially triethylsilane and triisopropyl-silane. Preferred are commercially available selenides.

A suitable silane is, for example, silane which is trisubstituted by a substituent selected from the group consisting of C₁-C₁₂-alkyl, especially C₁-C₇-alkyl, and C₂-C₃₀-acyl, especially C₁-C₈-acyl. Preferred are commercially available silanes.

The reductive amination is preferably carried out under acidic, neutral or preferably under basic conditions. A suitable base comprises, for example, an alkali metal hydroxide or carbonate, such as sodium hydroxide, potassium hydroxide or potassium carbonate. Furthermore, an amine base can be used, for example, tri-C₁-C₇-alkylamine, such as triethylamine, tri-n-propylamine, tri-butylamine or ethyl-diisopropylamine, a piperidine, such as N-methylpiperidine, or a morpholine, such as N-methyl-morpholine. Preferred bases include lithium hydroxide, sodium hydroxide, sodium hydrogencarbonate, sodium carbonate, potassium hydrogencarbonate and potassium carbonate. Especially preferred is sodium hydroxide, sodium carbonate or tri-n-propylamine.

The reductive amination is carried out in a suitable inert solvent or a mixture of solvents including water. Inert solvents conventionally do not react with the corresponding starting materials of formulae (II a) and (11 b). If an alkali metal borohydride such as sodium borohydride or lithium borohydride; an earth alkali metal borohydride such as calcium borohydride; an alkali metal cyanoborohydride, such as sodium cyanoborohydride or lithium cyanoborohydride, is used as reducing agent, for example, a polar solvent, for example, an alcohol such as methanol, ethanol, isopropanol or 2-methoxyethanol, or glyme, is preferred. If an alkali metal tri-(C₁-C₇-alkoxy)-borohydride such as sodium trimethoxy-ethoxy-borohydride; a tetra-C₁-C₇-alkylammonium-(cyano)borohydride such as tetrabutylammonium-borohydride or tetrabutylammonium-cyanoborohydride, is used as reducing agent, for example, hydrocarbons, such as toluene, esters such as ethylacetate or isopropylacetate, ethers such as tetrahydrofuran or tert-butylmethylether are preferred. If hydrogen or a hydrogen donor is used as reducing system, each in the presence of a hydrogenation catalyst, a polar solvent is preferred. The reductive amination can also be carried out e.g. in a mixture of an organic solvent with water, both mono- and biphasic. In a biphasic system a phase transfer catalyst such as tetrabutylammoniumhalide, e.g. bromide, or benzyltrimethylammonium halide, e.g. chloride, may be added.

If R₁ and R₂ both represent a protecting group and if the compound of formula (IIb) is a free base, the presence of a base is not required. If, however, R₁ is hydrogen and R₂ is a protecting group, not more than a molar equivalent of a base may be added. In order to avoid racemisation, the reaction is preferably carried out by using less than an equimolar amount of a base. If R₁ and R₂ each are hydrogen, no racemisation is observed even if the reaction is carried out with equal or more than one equivalent of base under mild conditions, preferably at temperatures between -10°C and 20°C.

The present invention likewise relates to novel compounds of formula (II a) that can be used as intermediates for the manufacture of the compound of formula (I).

The present invention likewise relates to novel compounds of formula (II b) that can be used as intermediates for the manufacture of the compound of formula (I).

The reaction of a compound of formula (II a) with a compound of formula (II b) results in an intermediately formed imine (Schiff base) of formula (II c') 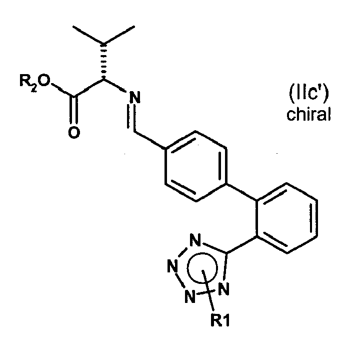 that can, under certain reaction conditions, be isolated or that can be subjected to the reduction without isolation.

The reductive amination is a two-step reaction, the formation of an imine by removing water, followed by the reduction step. The removal is an equilibrium reaction, which can be directed to the formation of an imine by continously eliminating the water, for example, by azeotropic removal. Furthermore, a water scavenger may be used to remove or inactivate free water which may be effected by a physical process such as absorption or adsorption or by a chemical reaction. A suitable water scavenger includes without limitation anhydrides of organic acid, aluminosilicates such as molecular sieves, other zeolites, finely divided silica gel, finely divided aluminia, anhydrides of inorganic acids such as phosphoric anhydride (P₂O₅), inorganic sulfates such as calcium sulfate, sodium sulfate, and magnesium sulfate, and other inorganic salts such as calcium chloride.

If Step (a) is carried out via first manufacturing and isolating a compound of formula (II c'), a compound of formula (II a) is reacted with a compound of formula (II b), maybe in the presence of a base, if R₁ and/or R₂ are hydrogen. Compounds of formula (II c') can then be converted into corresponding compounds of formula (II c) by reducing the compounds of formula (II c') with a corresponding reducing agent as mentioned above.

The intermediate imine of formula (II c') can, for example, be isolated by removal of the solvent, e.g. by distillation, especially by azeotropic removal of water.

In a preferred variant, the reductive amination is carried out without isolating a compound of formula (II c').

The reductive amination is most preferably carried out without removal of free water, especially, if R₁ and R₂ being hydrogen and with a base such as sodium hydroxide, a solvent such as methanol and a reducing reagent such as sodium borohydride.

In view of the imine structural element, compounds of formula (II c') comprise both the corresponding E and the corresponding Z isomer thereof. Preferred is the E isomer.

The present invention likewise relates to compounds of formula (II c') wherein R₁ is hydrogen or a tetrazole protecting group and wherein R₂ is hydrogen or a carboxy protecting group. Corresponding compounds can be used as intermediates for the manufacture of the compound of formula (I). Preferred are compounds of formula (II c'), wherein at least one of R₁ and R₂ represents hydrogen or both of R₁ and R₂ represent hydrogen.

The compounds of formulae (II a) and (II b) are partially known and can be prepared according to methods known per se.

Another embodiment of the present invention is a process for the manufacture of the compound of formula 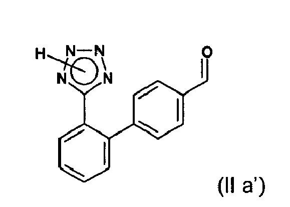 or a salt thereof, comprising
(i) reacting a compound of formula 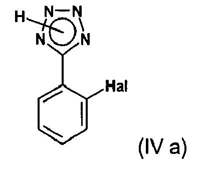 or a salt thereof, wherein Hal is halogen, with a compound of formula 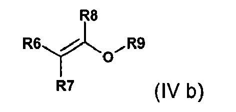 wherein R₆, R₇ and R₈, independently of one another, represent hydrogen or C₁-C₆-alkyl, such as methyl or ethyl, and R₉ represents C₁-C₆-alkyl, or R₇ and R₉ together form C₂-C₅- alkylene, such as ethylene, propylene, butylene, or R₆ and R₈ together form C₃-C₆-alkylene, in the presence of an acid; and
(ii) reacting a resulting compound of formula 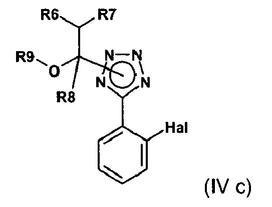 with a compound of formula 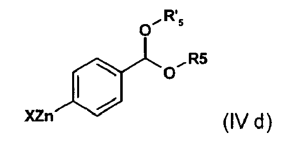 wherein X represents halogen such as iodine, bromine or chlorine, and R₅ and R'₅, independently of one another, represent C₁-C₇-alkyl, such as methyl or ethyl, or together form C₂-C₄-alkylene, such as ethylene, propylene, butylene or 1,2-dimethylethylene or 2,2-dimethylpropylene, in the presence of a transition metal catalyst; and
(iii) removing sequentially or in a single step the protecting groups from a resulting compound of formula 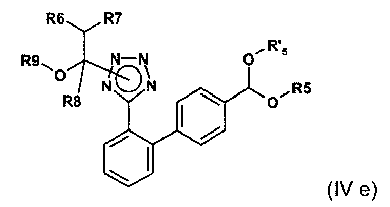 by treatment with an acid, preferably in the presence of water,
(iv) a resulting compound of formula (II a') or a salt thereof.

The reactions Steps (i) -(iv) described above in the variants are carried out, for example, in the absence or, customarily, in the presence of a suitable solvent or diluent or a mixture thereof, the reaction, as required, being carried out with cooling, at room temperature or with warming, for example in a temperature range from about -80°C up to the boiling point of the reaction medium, preferably from about -10°C to about +200°C, and, if necessary, in a closed vessel, under pressure, in an inert gas atmosphere and/or under anhydrous conditions.

Step (i) is carried out, for example, the in the presence of 0.0001 to 0.1 equivalents, preferably 0.001 to 0.04 equivalents of a Bronstedt acid, such as sulfuric acid, hydrochloric acid, phosphoric acid, methanesulfonic acid, ethanesulfonic acid, para-toluenesulfonic acid, camphor-10-sulfonic acid, trifluoroacetic acid, trichloroacetic acid, O,O'-dibenzoyltartaric acid and the like.

The reaction is carried out in a solvent which is sufficiently stable under anhydrous acidic conditions, e. g. in ethyl acetate, isopropyl acetate, an aromatic solvent, such as toluene or xylene, or an ethereal solvent, such as tert-butyl methyl ether, tetrahydrofuran, butyl ether or 1,2-dimethoxyethane, or a nitrile, such as acetonitrile. A preferred solvent is toluene.
The reaction temperature is between 15°C and the boiling point of the reaction medium, preferably between 30 and 60°C.

Step (ii) is carried out, for example, by using a conventional transition metal catalyst, for example, corresponding conventionally used platinium or palladium catalyst palladium, such as dichlorobis(triphenylphosphine)palladium(II).

Step (iii) carried out, for example, by dissolving a resulting compound of formula (IV e) in water or a mixture of water and an appropriate organic solvent and subsequent treating with an acid at elevated temperature. Crystallization of the product is accomplished by distilling off all or a part of the organic solvent, adding water, cooling the mixture or a combination of these measures. Appropriate organic solvents are ethers, such as tetrahydrofuran, 1,4-dioxan, butyl ether, nitriles, such as acetonitrile, alcohols, such as methanol, ethanol, 1-propanol, 2-propanol, isopropyl acetate, toluene, xylene, acetic acid or formic acid. Preferred solvents are methanol and ethanol. Suitable acids are Bronsted acids, such as sulfuric acid, hydrochloric acid, phosphoric acid, methanesulfonic acid, ethanesulfonic acid, para-toluenesulfonic acid, benzoic acid, acetic acid, formic acid. Preferred acids are sulfuric acid and hydrochloric acid. The amount of acid used is between 0.05 and 6.0 equivalents with respect to the starting material, preferably between 0.1 and 1.5 equivalents.

The isolation Step (iv) is carried out according to conventional isolation methods, such as by crystallizing the resulting compound of formula (II a') from the reaction mixture - if desired or necessary after work-up, especially by extraction - or by chromatography of the reaction mixture.

The compounds of formula (IV d) are prepared by reacting a compound of formula 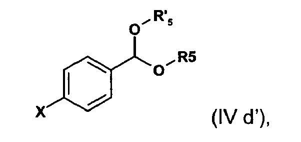 wherein X is halogen, for example, bromo, with magnesium under Grignard conditions, especially under anhydrous conditions, preferably in the presence of an activator such as 1,2-dibromo-ethane, to form a copmpound of formula 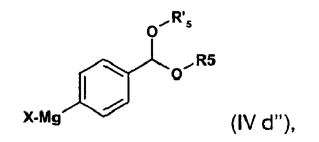 which is then treated with Zn(X)₂ , X being halogen, especially chloro.

As mentioned in the beginning of the specification, most of the angiotensin II receptor antagonists comprise as structural feature the tetrazole ring. In process sequences for the manufacture of such compounds the use of a protecting group for the tetazole ring is required.

For example, the triphenylmethyl group to protect the tetrazole ring against organometallic reagents is used. The triphenylmethyl group is cleaved later under acidic conditions. A disadvantage of the triphenylmethyl group is regarded to be its high molecular weight, which The 2-phenyl-2-propyl tetrazole protecting group is used to enable a metallation prior to any further conversion. The removal of this protecting group requires corrosive and toxic reagents, such as boron trifluoride etherate, or a transition metal catalyzed deprotection step, which are regarded as a disadvantage.

The protection of a tetrazole ring against organometallic reagents by using a 2-methyl-2-propyl group is another variant. For its removal this group requires harsh acidic conditions which are not compatible with sensitive functional groups in the compound or product.

Alternatively, the 2-cyanoethyl tetrazole protecting group is being used. The low stability of this protecting group towards most organometallic reagents, and the formation of highly toxic byproducts during deprotection are regarded as disadvantages.

Tetrazole rings can also be protected by a (phenylmethyl)oxymethyl group. However, one type of the two isomers formed is not stable towards organometallic reagents, not even at low temperatures.

The objective of the present invention is to provide a synthesis for the compound of formula (II a') or salts thereof by using protective groups which (1) do not have the disadvantages described above, (2) are easily introduced in high yield, (3) are of low weight, (4) are stable in the presence of organometallic reagents, such as aryl zinc and aryl magnesium compounds, (5) are easily removed in high yield under acidic conditions which are compatible with sensitive functional groups, such as a formyl group.

It has surprisingly been found that the process as described immediately above meets the above objectives. For example, a person skilled in the art would not expect that compounds of formula (IV c) can be used for the coupling with a compound of formula (IV d), as corresponding protecting groups of the tetrazole ring of compounds of formula (IV d) were not considered to stable in such a metal organic coupling reaction. A person skilled in the art would expect that corresponding protection groups of the tetrazoles of formula (IV d) would be split off. Furthermore, a person skilled in the art would expect that corresponding protection groups of the tetrazoles of formula (IV e) would be split off that easily under the mild conditions as outlined above.

Accordingly, another embodiment of the present invention are novel compounds of formulae (IV a), (IV b), (IV c), (IV d), (IV d'), (IV d"), and (IV e), especially compounds of formula (IV e).

A preferred embodiment of this aspect of the present invention relates to a compound of formula 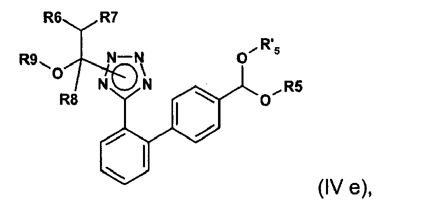 wherein R₅ and R'₅, independently of one another, represent C₁-C₇-alkyl, such as methyl or ethyl, or together form C₂-C₄-alkylene, such as ethylene, propylene, butylene or 1,2-dimethylethylene or 2,2-dimethylpropylene, or wherein R₆, R₇ and R₈, independently of one another, represent hydrogen or C₁-C₇-alkyl, such as methyl or ethyl, and R₉ represents C₁-C₇-alkyl, or R₇ and R₉ together form C₂-C₅-alkylene, such as ethylene, propylene, butylene, or R₆ and R₈ together form C₃-C₆-alkylene.

Preferred compounds of formula (IV e) are those compounds, wherein R₅ and R'₅, independently of one another, represent C₁-C₄-alkyl, such as methyl or ethyl, or together form C₂-C₄-alkylene, such as ethylene, propylene, butylene or 1,2-dimethylethylene or 2,2-dimethylpropylene, or wherein R₆, R₇ and R₈, independently of one another, represent hydrogen or C₁-C₄-alkyl, such as methyl or ethyl, and R₉ represents C₁-C₄-alkyl, or R₇ and R₉ together form C₂-C₅-alkylene, such as ethylene, propylene, butylene.

Even more preferred compounds of formula (IV e) are compounds wherein R₅ and R'₅, independently of one another, represent C₁-C₃-alkyl, such as methyl, ethyl or propyl, or wherein R₆, R₇ and R₈ represent hydrogen, and R₉ represents C₁-C₄-alkyl.

Most preferred compounds of formula (IV e) are compounds wherein R₅ and R'₅, independently of one another, represent C₁-C₃-alkyl, such as methyl, ethyl or propyl, and wherein R₆ and R₈ represent hydrogen, and R₇ and R₉ together form C₂-C₃-alkylene, such as ethylene or propylene.

Especially preferred are the compounds of formula (IV e) that are specifically described in the working examples.

In another embodiment of the invention, reaction Step (a) can be combined with the formation of a compound of formula (II a) with the conventional oxidation of a corresponding hydroxymethyl derivative of formula 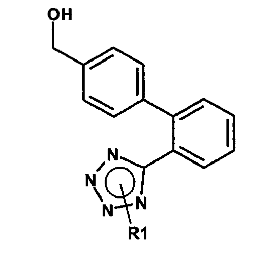 with the conventional reduction of a corresponding derivatives of the carboxylic acid of formula 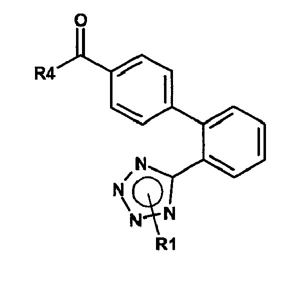 wherein R₄ represents, for example, hydroxy, C₁-C₇-alkoxy or halogen such as chloro; or with the conventional hydrolysis of an acetale of formula 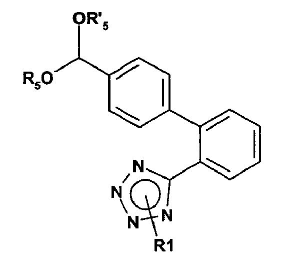 wherein R₅ and R₅', independently of one another, represent C₁-C₇-alkyl, such as methyl or ethyl, or together form C₂-C₄-alkylene, such as ethylene, propylene or butylene or 1,2-dimethylethylene.

The present invention likewise relates to reaction Step (a), especially the reduction step of reductive amination. If the reaction is carried out, for example, with a borohydride and under basic conditions in a polar solvent, optionally, in the presence of water, preferably in a lower (especially anhydrous) alkanol such as methanol, ethanol, isopropanol or glyme, the resulting compound of formula (II c) or (II c'), respectively, can surprisingly be obtained in an essentially enantiomerically pure form. It is expected that under basic conditions, normally an at least partial racemisation will take place. In contrast to this, surprisingly, for example, an enantiomer excess (ee) of a compound of formula (II c) or (II c'), respectively, of ≥ 95%, preferably ≥ 98% and most preferably ≥ 99% can be achieved.

Step (a) is preferably carried out under mild conditions, especially in a temperature range of about -10°C to about room temperature, preferable in a range of about -5°C and +5°C.

### Step (b):

In reaction Step (b), the acylation is carried out, for example, in absence or in presence of a suitable base.

Suitable bases are, for example, alkali metal hydroxides or carbonates, morpholine or piperidine amines, unsubstituted or substituted pyridines, anilines, naphthalene amines, tri-C₁-C₇-alkylamines, basic heterocycles or tetra-C₁-C₇-alkyl-ammonium hydroxides. Examples, which may be mentioned, include sodium hydroxide, potassium carbonate, triethylamine, tri-propyl-amine, tri-butyl-amine or ethyldiisopropylamine, N-methyl-morpholine or N-methyl-piperidine, dimethylaniline or dimethylamino-naphthalene, a lutidine, a collidine, or benzyltrimethylammonium hydroxide. A preferred base is a tri-C₁-C₄-alkylamine such as ethyl-diisopropyl-amine or is pyridine.

The acylation is carried out in a suitable inert solvent or in a mixture of solvents. The person skilled in the art is fully enabled to select a suitable solvent or solvent system. For example, an aromatic hydrocarbon such as toluene, an ester such as ethylacetate or a mixture of ethylacetate and water, a halogenated hydrocarbon such as methylene chloride, a nitrile such as acetonitrile of proprionitrile, an ether such as tetrahydrofurane or dioxane, 1,2-dimethoxy-ethane, amide such as dimethylformamide, or a hydrocarbon, such as toluene, is used as solvent.

During the acylation of a compound of formula (II c), if R₂ is hydrogen, the carboxyl-group might also be acylated forming a mixed anhydride. This intermediate is strongly prone to racemisation, mainly under basic conditions. Racemisation however can be avoided by first adding the compound of formula (II d), especially the halide, to the compound of formula (IIc) in a suitable inert solvent (e.g. dimethoxyethane, tetrahydrofuran or acetonitril), then slowly adding a sub-stoichiometric amount of the base, especially pyridine, in relation to the compound of formula (II d). Small amounts of water in the reaction mixture, preferably two equivalents, may additionally suppress racemisation.

The reaction can also be carried out by simultaneous or alternative addition of a compound of formula (II d) and a base such as pyridine keeping the reaction mixture acidic at all times.

The invention likewise relates to a compound of formula (II c), wherein R₁ is hydrogen or a tetrazole protecting group and R₂ is hydrogen or a carboxy protecting group, excluding a compound of formula (II c), wherein R₁ is ethyl and R₂ is trityl; that can be used e.g. as intermediate for the manufacture of the compound of formula (I).

The invention likewise relates to reaction Step (b). The resulting compound of formula (II e) can be obtained in an essentially enantiomerically pure form. For example, an enantiomer excess (ee) of a compound of formula (II c) or (II c'), respectively, of ≥ 95%, preferably ≥ 98% and most preferably ≥ 99% can be achieved.

If R₂ represents a protecting group and R₁ is hydrogen or a protecting group, for example, two equivalents of both a compound of formula (II d), e.g. the corresponding halide thereof, and a base, e.g. ethyl-diisopropyl-amine or tri-n-propylamine are added to a corresponding compound of formula (II c) dissolved in a suitable solvent, e.g. toluene. Surprisingly, no racemisation is observed.

Normally, in corresponding compounds of formula (II c), wherein R₂ is hydrogen or a protecting group, one would expect at least partial racemisation, mainly in presence of base or acid and at elevated temperature. However, no racemisation is observed under the conditions applied according to this invention.

Normally, in corresponding compounds of formula (II c), wherein R₂ is hydrogen, one would expect a racemisation. However, in a presence of a base, no racemisation is observed.

If R₁ is hydrogen and R₂ a protecting group, the tetrazole ring might also be acylated. When, however, the reaction mixture is quenched, for example with water or an alcohol such as methanol, the corresponding compound can be obtained wherein R₁ is hydrogen.

Compounds of formula (II d) are known or can be manufactured according to methods known per se.

### Step (c):

The removal of the protecting groups, both the tetrazole and carboxy protecting group, can be carried out according to methods known per se in the art.

For example, a benzylester can be converted into the corresponding acid especially by hydrogenation in the presence of a suitable hydrogenation catalyst. A suitable catalyst comprises, for example, nickel, such as Raney nickel, and noble metals or their derivatives, for example oxides, such as palladium or platinum oxide, which may be applied, if desired, to support materials, for example to carbon or calcium carbonate. The hydrogenation may preferably be carried out at pressures between 1 and about 100 atm. and at room temperature between about -80° to about 200°C, in particular between room temperature and about 100°C.

The removal of a trityl or tert-butyl group, respectively, can be achieved by treating corresponding protected compounds with an acid, especially under mild conditions.

### Step (d):

The isolation Step (d) of a compound of formula (I) is carried out according to conventional isolation methods, such as by crystallizing the resulting compound of formula (I) from the reaction mixture - if desired or necessary after work-up, especially by extraction - or by chromatography of the reaction mixture.

The conversion of an acid of formula (I) into a salt is carried out in a manner known per se. Thus, for example, a salt with a base of compounds of the formula (I) is obtained by treating the acid form with a base. Salts with a base can, on the other hand, be converted into the acid (free compound) in a customary manner, and salts with a base can be converted, for example, by treating with a suitable acid agent.

The present invention likewise relates to the novel compounds as described in the Working Examples part.

The following examples illustrate the invention described above; however, they are not intended to limit its extent in any manner.

### Working Examples:

### Example 1:

### a) Preparation of 3-methyl-2{[1-[2'-(1H-tetrazol-5-yl)-biphenyl-4-yl]-meth-(E/Z)-ylidene]-amino}-butyric acid

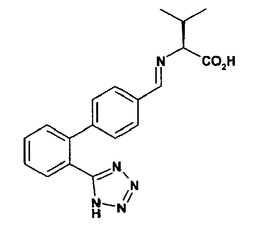

Aqueous sodium hydroxide solution 30% (4.2 ml; 31.5 mmol) is added to a stirred suspension of L-Valine (2.43 g; 20.8 mmol) and 2'-(1H-tetrazol-5-yl)-biphenyl-4-carbaldehyde (5 g; 19.6 mmol), in water (20 ml) at room temperature, until pH 11 is reached. The resulting solution is stirred at room temperature for 15 minutes. The clear solution is evaporated at 60°C in vacuo, and remaining water is azeotropically removed with 10 ml 1-butanol.
¹H NMR (CD₃OD, 300MHz):
δ = 8.21 (CH=N, s), 7.67 (C₆H₅-CH, d), 7.40-7.60 (4 C₆H₅-CH, m), 7.18 (C₆H₅-CH, d), 3.42 (CH, d), 2.31 (CH, m), 0.98 (CH₃, d), 0.82 (CH₃, d).

### b1) Preparation of (S)-3-Methyl-2-((2'-(1H-tetrazol-5-yl)-biphenyl-4-ylmethyl)-amino)-butyric acid

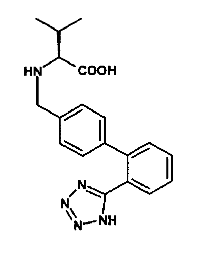

Aqueous sodium hydroxide solution 2.0 M (approximately 100 ml; 200 mmol) is added to a stirred suspension of L-Valin (11.8 g; 100 mmol) and 2'-(1H-tetrazol-5-yl)-biphenyl-4-carbaldehyde (25.1 g; 100 mmol) in water (100 ml) at room temperature, until pH 11 is reached. The resulting clear solution is evaporated at 60°C in vacuo, and remaining water is azeotropically removed with 1-butanol. The residue (imine as a solid foam) is dissolved in absolute ethanol (300 ml), and sodium borohydride (3.78 g; 100 mmol) is added in portions to the solution at 0-5°C. The reaction mixture is stirred for 30 min at 0-5°C, and, it the reaction is complete (HPLC), quenched by addition of water (100 ml) and hydrochloric acid 2.0 M (80 ml; 160 mmol). The organic solvent (ethanol) is stripped off from the clear solution (pH 7) at 50°C in vacuo. The remaining aqueous concentrate is adjusted to pH 2 by slow addition of hydrochloric acid 2.0 M (approximately 70 ml; 140 mmol) at 40°C. During the addition the desired product precipitates. It is collected by filtration, washed with water and dried in vacuo. The crude product is suspended in methanol at 50°C, and the slurry is cooled to room temperature. (S)-3-methyl-2-((2'-(1H-tetrazol-5-yl)-biphenyl-4-yl-methyl)-amino)-butyric acid is collected by filtration and then dried in vacuo.

### b2) Alternatively, (S)-3-methyl-2-((2'-(1H-tetrazol-5-yl)-biphenyl-4-yl-methyl)-amino)-butyric acid can be prepared e.g. as follows:

Aqueous sodium hydroxide solution 10 M (approximately 41 ml; 410 mmol) is added to a stirred suspension of L-Valine (24.8 g; 210 mmol) and 2'-(1 H-tetrazol-5-yl)-biphenyl-4-carbaldehyde (50 g; 200 mmol) in water (200 ml) at room temperature, until pH 11 is reached. The resulting clear solution is evaporated at 60°C in vacuo, and remaining water is azeotropically removed with 1-butanol. The residue (imine as a solid foam) is dissolved in methanol (600 ml), and sodium borohydride (3.13 g; 80 mmol) is added in portions to the solution at 0-5°C. The reaction mixture is stirred for 30 min at 0-5°C, and, if the reaction is complete (HPLC), quenched by addition of water (300 ml) and hydrochloric acid 2.0 M (160 ml; 320 mmol). The organic solvent (methanol) is stripped off from the clear solution (pH 7) at 50°C in vacuo. The remaining aqueous concentrate is adjusted to pH 2 by slow addition of hydrochloric acid 2.0 M (approximately 90 ml) at 40°C. During the addition the desired product precipitates. It is collected by filtration, washed with water and dried in vacuo. The crude product is suspended in methanol at 50°C, and stirred for a few minutes. Then the slurry is cooled to room temperature. (S)-3-methyl-2-((2'-(1H-tetrazol-5-yl)-biphenyl-4-yl-methyl)-amino)-butyric acid is collected by filtration and then dried in vacuo.
Enantiomeric excess (by HPLC): ee > 99.9 %

### b3) Alternatively, (S)-3-methyl-2-((2'-(1H-tetrazol-5-yl)-biphenyl-4-yl-methyl)-amino)-butyric acid can be prepared e.g. as follows:

Sodium hydroxide (1.71g; 41.89 mmol) is added in portions to a stirred suspension of L-Valine (2.48 g; 21 mmol) in 15 ml methanol. The mixture is stirred at room temperature for 30 minutes. Then 2'-(1H-tetrazol-5-yl)-biphenyl-4-carbaldehyde (5 g; 20 mmol) is added. The mixture becomes a clear solution after a few minutes. The mixture is then cooled to -5°C and sodium borohydride (0.315 g; 8 mmol) is added in portions to the solution. The temperature is maintained between 0-5°C during the addition. The resulting mixture is stirred for 2 hours at 0°C - the reaction completion is followed by HPLC - then quenched by addition of water (10 ml) and hydrochloric acid 37% (5.3 g) until pH is between 2-2.5. Further work-up and crystallisation are done according to example 1 b2).
Enantiomeric excess (by HPLC): ee > 99.9 %

### b4) Alternatively, (S)-3-methyl-2-((2'-(1H-tetrazol-5-yl)-biphenyl-4-yl-methyl)-amino)-butyric acid can be prepared e.g. as follows:

In a 50 ml steel autoclave, 3-methyl-2{[1-[2'-(1 H-tetrazol-5-yl)-biphenyl-4-yl]-meth-(E/Z)-ylidene]-amino}-butyric acid (1.5 g; 3.2 mmol) and 5% Pt/C (7.5 mg, 5% wt/wt) is charged under argon. Then 15 ml methanol are added and the autoclave is sealed and flushed with argon and hydrogen. The pressure is set to 5 bars and the reaction stirred at room temperature. The reaction completion is monitored by HPLC. Then the autoclave is flushed with argon and the catalyst is filtered off. Further work-up and crystallisation are done similar to example 1 b2).

### b5) Alternatively, (S)-3-methyl-2-((2'-(1H-tetrazol-5-yl)-biphenyl-4-yl-methyl)-amino)-butyric acid can be prepared e.g. as follows:

2'-(1H-tetrazol-5-yl)-biphenyl-4-carbaldehyde (0.79 g; 3.2 mmol) and L-Valine (0.4 g; 3.4 mmol) are suspended in 15 ml methanol. Then sodium hydroxide is added (0.27 g; 6.72 mmol) and the reaction mixture is stirred at room temperature until a clear solution is obtained. 5% Pt/C (15.8 mg; 2 wt/wt-%) is added. The autoclave is sealed and flushed with argon and hydrogen. The pressure is set to 5 bars and the reaction is stirred at 60°C. The reaction completion is monitored by HPLC. Then the autoclave is flushed with argon and the catalyst is filtered off. Further work-up and crystallisation are done similar to example 1 b2).
Enantiomeric excess (by HPLC): ee > 99.9 %.

### c) Preparation of (S)-3-Methyl-2-{pentanoyl-[2'-(1H-tetrazol-5-yl)-biphenyl-4-ylmethyl]-amino}-butyric acid

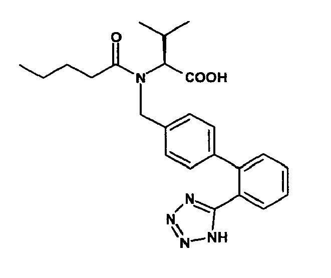

A suspension of (S)-3-methyl-2-((2'-(1H-tetrazol-5-yl)-biphenyl-4-ylmethyl)-amino)-butyric acid (17.6 g; 50.0 mmol) in 1,2-dimethoxyethan (116 g) is cooled to -5°C, and valeroylchloride (9.9 ml; 80 mmol) is added, followed by slow addition of pyridine (6.0 ml; 75 mmol) diluted with 1,2-dimethoxyethane (60 ml). [1] After completion of the reaction, the reaction mixture is quenched with methanol (18 ml). Finally water (50 ml) is added at room temperature, and after stirring for 1 h, the mixture is adjusted to pH 7.5 by addition of aqueous sodium carbonate solution 10% (- 116 ml, 120 mmol) at 0°C. The organic solvents are stripped off at 50°C in vacuo. Ethylacetate (125 ml) is added to the remaining aqueous concentrate, and the two-phase system is adjusted to pH 2 at 0-5°C by addition of 2.0 M HCl (- 98 ml). The organic phase is separated and concentrated at 45°C in vacuo (water is azeotropically removed). The crystallization of the product is initiated at 45°C and - after addition of cyclohexan (102 ml) - completed by cooling to -5°C. The solid is collected by filtration, and after drying at 50°C, (S)-3-methyl-2-{pentanoyl-[2'-(1H-tetrazol-5-yl)-biphenyl-4-ylmethyl]-amino} -butyric acid is received as a white powder.
Melting point: 108-110°C
Enantiomeric excess (by HPLC): ee > 99.5 %
[1] Alternatively pyridine and valeroylchloride can be added alternately: A suspension of (S)-3-methyl-2-((2'-(1H-tetrazol-5-yl)-biphenyl-4-ylmethyl)-amino)-butyric acid (25.5 g; 72.6 mmol) in 1,2-dimethoxyethane (126 g) is cooled to -10°C, and valeroylchloride (8.75 g; 72.6 mmol) is added over 15 min., followed by slow addition of a mixture (7.16 g) of pyridine (5.6 g) and water (1.5 g) over 61 min. After stirring for 30 min. valeroylchloride (5.3 g; 43.5 mmol) is added over 8 min., followed by slow addition over 30 min. of a mixture (4.3 g) of pyridine (3.4 g) and water (0.9 g). After each addition of pyridine the pH is controlled by sampling (hydrolyzed with water). The pH of the samples should always be below 2.5. The reaction is stirred for 25 min., then water (25.6 g) is added over 30 min. The mixture is stirred for another 30 min., then warmed to 23°C over 30 min. and stirred for another 2 hours. Adjustement of pH, remove of organic solvents by distillation, further work-up and crystallization is done as described in the example 1 c) above.

### Example 2:

This example can be illustrated by means of the following reaction scheme:

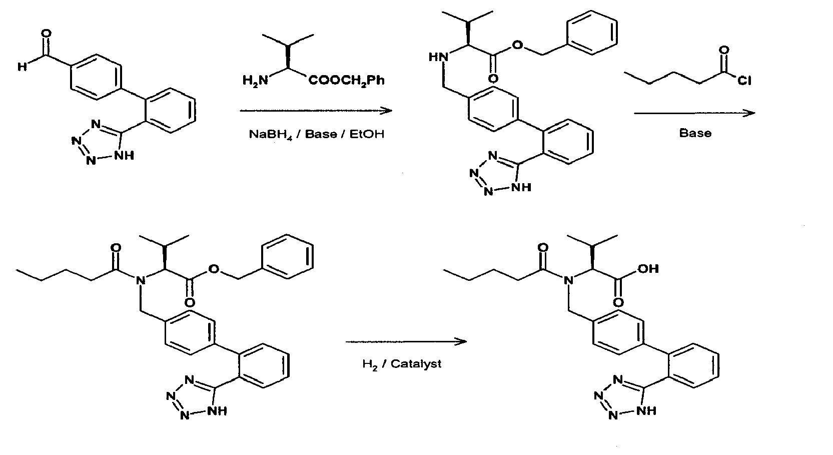

### a) Preparation of (S)-3-Methyl-2-{[2'-(1H-tetrazol-5-yl)-biphenyl-4-yl-methyl]-amino}-butyric acid benzylester

L-Valin-benzylester tosylate (6.38 g, 16.8 mmol) in toluene (40 ml) is extracted with a solution of sodiumcarbonate (2.36 g, 22.0 mmol) in water (40 ml). The organic phase (contains L-valin benzylester free base is separated, and 2'-(1 H-tetrazol-5-yl)-biphenyl-4-carbaldehyde (4.13 g, 16.0 mmol) and tri-n-propyl-amine (3.20 ml, 16.8 mmol) are added at room temperature. The resulting solution is evaporated at 50°C in vacuo (water is removed azeotropically). The residual oil (containing the intermediate imine is dissolved in absolute ethanol (40 ml), and sodium borohydride (0.68 g, 17.6 mmol) is added in portions within 10 minutes (min.) at 0-5°C. The resulting solution is stirred for 30 min at 0-5°C. After completion of the reaction, the reaction mixture is quenched with water (10 ml) and adjusted to pH 6-7 by addition of hydrochloric acid 2 M (16 ml, 32 mmol) at RT. Ethanol is distilled off from the reaction mixture at 50° in vacuo and the residual aqueous mixture extracted with toluene (60 ml). The organic phase is concentrated at 50°C in vacuo to approximately 50 % of the original volume by destillation (water and ethanol are azeotropically removed). The resulting concentrate (35 ml) containing (S)-3-methyl-2-{[2'-(1H-tetrazol-5-yl)-biphenyl-4-yl-methyl]-amino}-butyric acid benzylester is used as it is as starting material for the subsequent acylation step.

### b) Preparation of (S)-3-Methyl-{2-pentanoyl-[2'-(1H-tetrazol-5-yl)-biphenyl-4-yl-methyl]-amino}-butyric acid benzylester

The solution of (S)-3-methyl-2-{[2'-(1H-tetrazol-5-yl)-biphenyl-4-yl-methyl]-amino}-butyricacid benzylester (approximately 7.0 g , 16.0 mmol) in toluene (35 ml) from the previous step is diluted with toluene (35 ml). The clear solution is cooled to 0-5°C under anhydrous conditions, and N-ethyl-diisopropylamine (6.1 ml, 35.2 mol) and valeroylchloride (4.1 ml, 33.6 mmol) are added at this temperature. The reaction mixture is heated to 50°C within 30 min and agitated at 50°C for approximately 1 h and - after completion of the reaction - quenched by addition of methanol (10 ml) at 50°C. The clear solution is stirred for approximately 30 min at 50°C and finally cooled to RT. Water (30 ml) is added and the resulting two-phase system is adjusted to pH 2 by addition of hydrochloric acid 2.0 M (approximately 11 ml, 22 mmol). The organic phase is separated, extracted with water (30 ml) and concentrated at 50°C in vacuo to approximately 50 % of the original volume by destillation (water and methanol are azeotropically removed). The resulting concentrate in toluene (40 ml) is seeded at 40°C in order to start the crystallization and agitated at this temperature for approximately 1 hour (h). The suspension is gradually cooled to 0°C within 6-10 h. The solid is separated by filtration, washed with cold toluene (30 ml) and dried in vacuo at 50°C to give (S)-3-methyl-{2-pentanoyl-[2'-(1H-tetrazol-5-yl)-biphenyl-4-yl-methyl]-amino}-butyric acid benzylester.
Melting point: 115 - 116°C
Enantiomeric excess (by HPLC): ee > 99.8 %

### c) Preparation of (S)-3-Methyl-2-{pentanoyl-[2'-(1H-tetrazol-5-yl)-biphenyl-4-yl-methyl]-amino}-butyric acid

A solution of (S)-3-Methyl-{2-pentanoyl-[2'-(1H-tetrazol-5-yl)-biphenyl-4-yl-methyl]-amino}-butyric acid benzylester (10.6 g; 20.0 mmol) in Ethylacetate (43 ml) is hydrogenated at 4 bar / 50°C in presence of a wet palladium on charcoal catalyst 5% (1.12 g, containing 50 % water). After completion of the reaction (cease of hydrogen consumption) the catalyst is removed by filtration and the filtrate is concentrated at 45°C in vacuo (water is azeotropically removed). The crystallization of the product is initiated at 45°C and - after addition of cyclohexan (102 ml) - completed by cooling to -5°C. The solid is collected by filtration, and after drying at 50°C, (S)-3-methyl-2-(pentanoyl-[2'-(1H-tetrazol-5-yl)-biphenyl-4-yl-methyl]-amino)-butyric acid is received as a white powder.
Melting point: 108-110°C.
Enantiomeric excess (by HPLC): ee > 99.5 %

### Example 3:

### a) Preparation of (S)-3-Methyl-2-((2'-(1H-tetrazol-5-yl)-biphenyl-4-yl-methyl)-amino)-butyric acid tert-butylester

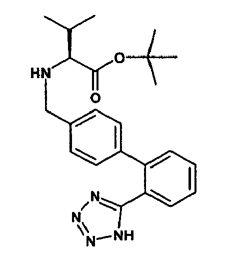

To a suspension of L-valine tert-butylester hydrochloride (419.4 mg; 2 mmol) in 5 ml of isopropylacetate is added sodium carbonate (265 mg; 2.5 mmol) in 5 ml of water. After complete dissolution, the two phases are separated immediately. The aqueous layer is washed once with 4ml of isopropylacetate. The combined organic layers are washed with 5 mL of water. The colourless organic is dried over sodium sulfate, filtered, evaporated in vacuum and dried in high vacuum to give a colourless oil. The oil is dissolved in 4 ml of methanol. After addition of the 2'-(1 H-tetrazol-2-yl)-biphenyl-4-carbaldehyde (515 mg; 2mmol) and triethylamine (0.278 ml; 2 mmol), the yellow solution is stirred for 5 minutes before evaporation in vacuum to give a yellow oil. After dissolution in 4 ml ethanol the solution is cooled to 0°C. Sodiumborohydride (78 mg; 2 mmol) is added in 4 portions with stirring until the imine dissapeared (HPLC). The slightly yellow solution is acidified from pH 11 to pH 6 with 3.2 ml of a 1.0 M HCl solution. Evaporation of the ethanol affords a mixture of a yellow oil in water. This mixture is extracted with isopropylacetate. The combined organic layers are dried over sodium sulfate, filtered, evaporated in vacuum and dried in high vacuum to give (S)-3-methyl-2-((2'-(1H-tetrazol-5-yl)-biphenyl-4-ylmethyl)-amino)-butyric acid tert-butylester as an oil.

### b) Preparation of (S)-3-Methyl-{2-pentanoyl-[2'-(1 H-tetrazol-5-yl)-biphenyl-4-yl-methyl]-amino}-butyric acid tert-butylester

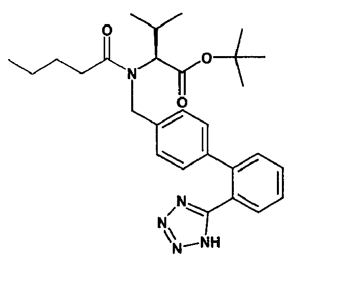

(S)-3-methyl-2-((2'-(1H-tetrazol-5-yl)-biphenyl-4-ylmethyl)-amino)-butyric acid tert-butylester (8.5 g; -16.0 mmol) is dissolved in toluene (63 ml) and N-ethyl-diisopropylamine (6.1 ml; 35.2 mmol) and valeroylchloride (4.1 ml; 33.6 mmol) are added at RT. The clear solution is heated to 50°C and stirred at this temperature for 60 min. After completion of the reaction, the reaction mixture is quenched with methanol (10 ml) at 50°C, and finally, water is added at RT. The two-phase system is adjusted to pH 2 by addition of 2.0 M HCl (- 5 ml). The organic phase is separated and concentrated at 50°C in vacuo (remaining water is removed azeotropically). On cooling to RT, the product starts to crystallize from toluene. (S)-3-methyl-{2-pentanoyl-[2'-(1 H-tetrazol-5-yl)-biphenyl-4-yl-methyl]-amino}-butyric acid tert-butylester is received as a white powder after filtration and drying in vacuo.
Melting point: 153.4°C
Enantiomeric excess (by HPLC): ee > 99.8 %

### Example 4:

### a) Preparation of (S)-2-((2'-(2"-tert-Butyl-tetrazol-5"-yl)-biphenyl-4-ylmethyl)-amino)-3-methyl-butyric acid

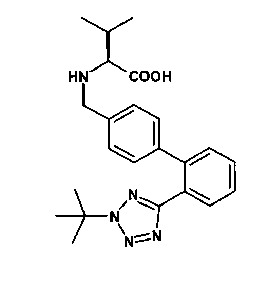

A soda solution (1 mol/l; 1.0 ml, 1.0 mmol) is added to L-valine (117.15 mg; 1.0 mmol). After complete dissolution, the reaction is evaporated. To the white solid is added 2'-(1 H-tert-butyl-tetrazol-2-yl)-biphenyl-4-carbaldehyde (306.4 mg, 1 mmol) and 4 ml of methanol. After complete dissolution the reaction is evaporated and the slightly yellow oil is dried in high vacuum. The imine is dissolved in 4 ml ethanol and cooled to 0°C before sodium borohydride (38 mg; 1.0 mmol) is added in 2 portions with stirring until the imine disappeared. The slightly yellow solution is acidified with 1,8 ml of a 1 N HCl solution to pH 6-7. Evaporation in vacuum affords a white solid. 10 ml of isopropylacetate and 10mL water are added. The white precipitate is filtered, washed with water and dried to result in 2-((2'-(2"-tert-butyl-tetrazol-5"-yl)-biphenyl-4-yl-methyl)-amino)-3-methyl-butyric acid.
Melting point: 189.7°C

### Example 5:

### a) Preparation (S)-2-{[2'-(2-Benzyl-2H-tetrazol-5-yl)-biphenyl-4-ylmethyl]amino}-3-methyl-butyric acid benzyl ester

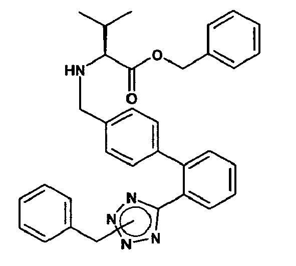

L-Valin-benzyl ester tosylate (0.97 mmol, 368 mg) is suspended in isopropylacetate (4 ml). To this suspension was added a solution of sodium carbonate (1.21 mmol, 128 mg) in water (2 ml) at room temperature. The resultant mixture is agitated for 2 minutes, transferred to a separating funnel and the phases separated. The organic phase is dried over sodium sulphate, filtered and concentrated in vacuo to afford the free base as a colourless oil. The 2'-(1H-benzyl-tetrazol-2-yl)-biphenyl-4-carbaldehyde (0.88 mmol, 300 mg) is dissolved in 1,2 dimethoxyethane (4 ml) at room temperature and the resultant solution added to the free base residue. After 8 hours the solvent is removed in vacuo and the residue dissolved in ethanol (4 ml). Sodium borohydride (1.1 mmol, 41.6 mg) is added to the reaction mixture. The resultant opaque solution is stirred at room temperature for more than 2 hours and then concentrated in vacuo to remove the ethanol. Water (20 ml) and dichloromethane (20 ml) are added and the aqueous phase pH is adjusted to 1 by addition of 1 N HCl. The phases are separated and the aqueous phase extracted again with dichloromethane (10 ml). The combined organic phases are washed with water (10 ml), dried over anhydrous sodium sulphate, filtered and concentrated in vacuo to afford the title compound as a colourless oil.

### Example 6:

### a) Preparation of (S)-2-{[2'-(2-tert-Butyl-2H-tetrazol-5yl)-biphenyl-4-ylmethyl]-amino}-3 methyl-butyric acid tert-butyl ester

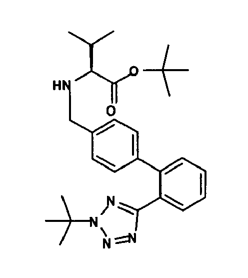

L-Valin-t-butyl ester hydrochloride (1.32 mmol, 278 mg) is suspended in isopropylacetate (5 ml). To this suspension was added a solution of sodium carbonate (1.65 mmol, 175 mg) in water (5 ml) at room temperature. The resultant mixture is agitated for 2 minutes, transferred to a separating funnel and the phases separated. The organic phase is dried over sodium sulphate, filtered and concentrated in vacuo to afford the free base as a colourless oil.

The 2'-(1H-tert.-butyl-tetrazol-2-yl)-biphenyl-4-carbaldehyde (1.2 mmol, 367.2 mg) is dissolved in ethanol (5 ml) at room temperature and the resultant solution added to the free base residue. After 90 minutes sodium borohydride (1.5 mmol, 56.7 mg) is added to the reaction mixture. The resultant opaque solution is stirred at room temperature for 2 hours and then concentrated in vacuo to remove the ethanol. Water (20ml) and dichloromethane (20 ml) are added and the aqueous phase pH is adjusted to 1 by addition of 1 N HCl. The phases are separated and the aqueous phase extracted again with dichloromethane (10 ml). The combined organic phases are washed with water (10 ml), dried over anhydrous sodium sulphate, filtered and concentrated in vacuo to afford the title compound as a colourless oil.
¹H NMR (CD₃OD, 400MHz):
δ =7.86 (1 H, d, J=8 Hz), 7.41-7.68 (3H, m), 7.44 (2H, d, J=8Hz), 7.24 (2H, d, J=8Hz), 4.17 (1 H, d, J=13Hz), 4.08 (1H, d, J=13Hz), 3.56 (1 H, d, J=2Hz), 2.27 (1 H, m), 1.12 (3H, d, J=7Hz) and 1.06 (3H, d, J=7Hz).

### Example 7:

### a) Preparation of (S)-3-Methyl-2-{[2'-(1H-tetrazol-5-yl)-biphenyl-4-yl-methyl]-amino}-butyric acid benzylester

L-Valin-benzylester tosylate (20.1 g, 53 mmol) in toluene (90 ml) is extracted with a solution of sodiumcarbonate (7.3 g, 69 mmol) in water (125 ml). The organic phase (contains L-valin benzylester free base) is separated, and 2'-(1H-tetrazol-5-yl)-biphenyl-4-carbaldehyde (12.5 g, 50 mmol) and N-ethyl-diisopropylamine (9.0 ml, 52 mmol) are added at room temperature. The resulting solution is completely evaporated at 50°C in vacuo (water is removed azeotropically). The residual oil (containing the intermediate imine) is dissolved in methanol (160ml), and sodium borohydride (0.84 g, 22 mmol) is added in portions within 10 min at 0-5°C. The resulting solution is stirred for 30 min at 0-5°C. After completion of the transformation, the reaction mixture is quenched by addition of 1.0 M hydrochloric acid (approximately 42 ml, 42 mmol) at 0-5°C and adjusted to pH 6-7. Methanol is distilled off from the reaction mixture at 50°C in vacuo and the residual aqueous mixture extracted with toluene (180 ml). The organic phase is concentrated at 50°C in vacuo to approximately 50% of the original volume by distillation (water and methanol are azeotropically removed). The resulting concentrate (approximately 80 g) containing (S)-3-methyl-2-{[2'-(1H-tetrazol-5-yl)-biphenyl-4-yl-methyl]-amino}-butyric acid benzylester is used as it is as starting material for the subsequent acylation step.

### b) Preparation of (S)-3-Methyl-{2-pentanoyl-[2'-(1H-tetrazol-5-yl)-biphenyl-4-yl-methyl]-amino}-butyric acid benzylester

The solution of (S)-3-methyl-2-{[2'-(1H-tetrazol-5-yl)-biphenyl-4-yl-methyl]-amino}-butyric acid benzylester in toluene (approximately 80 g , 48-50 mmol) from the previous step is diluted with toluene (85 ml). Under anhydrous conditions, N-ethyl-diisopropylamine (24.0 ml, 140 mol) and valeroylchloride (17.3 ml, 140 mmol) are slowly added at 20°C internal temperature. The reaction mixture is agitated for approximately 30 min and - after completion of the transformation - quenched by addition of methanol (31 ml) at 20°C. The clear solution is agitated for 30 min at 20°C, then water (78 ml) is added and the resulting two-phase system is adjusted to pH 2 by addition of 2.0 M hydrochloric acid (approximately 10 ml, 20 mmol). The organic phase is separated, extracted with water (78 ml) and concentrated at 50°C in vacuo to approximately 50% of the original volume by distillation (water and methanol are azeotropically removed). The resulting concentrate in toluene (- 94 g) is seeded at 40°C in order to initiate the crystallization and agitated at this temperature for approximately 1 h. The suspension is gradually cooled to 0°C within 6-10 h. The solid is separated by filtration, washed with cold toluene (60 ml) and dried in vacuo at 50°C to give (S)-3-methyl-{2-pentanoyl-[2'-(1H-tetrazol-5-yl)-biphenyl-4-yl-methyl]-amino}-butyric acid benzylester.
Melting point: 115 - 116°C.
Enantiomeric excess (by HPLC): ee > 99.8 %.

### c) Preparation of (S)-3-Methyl-2-{pentanoyl-[2'-(1H-tetrazol-5-yl)-biphenyl-4-yl-methyl]-amino}-butyric acid

A solution of (S)-3-Methyl-{2-pentanoyl-[2'-(1H-tetrazol-5-yl)-biphenyl-4-yl-methyl]-amino}-butyric acid benzylester (10.6 g; 20.0 mmol) in ethylacetate (43 ml) is hydrogenated at 4 bar / 50°C in presence of a wet palladium on charcoal catalyst 5% (1.12 g, containing 50 % water). After completion of the reaction (cease of hydrogen consumption) the catalyst is removed by filtration and the filtrate is concentrated at 45°C in vacuo (water is azeotropically removed). The crystallization of the product is initiated at 45°C and - after addition of cyclohexan (102 ml) - completed by cooling to -5°C. The solid is collected by filtration, and after drying at 50°C, (S)-3-methyl-2-{pentanoyl-[2'-(1H-tetrazol-5-yl)-biphenyl-4-yl-methyl]-amino} - butyric acid is received as a white powder.
Melting point: 108-110°C.
Enantiomeric excess (by HPLC): ee > 99.5 %

### Example 8:

### 5-(2-Chlorophenyl)-2-(tetrahydropyran-2-yl)-2H-tetrazole and 5-(2-Chlorophenyl)-1-(tetrahydropyran-2-yl)-1H-tetrazole.

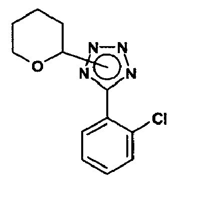

Methanesulfonic acid (0.141 g; 1.44 mmol) is added to a suspension of 5-(2-chlorophenyl)-1 H-tetrazole (88.46 g; 480.0 mmol) in toluene (660 ml). The resulting mixture is heated to 50°C and a solution of 3,4-dihydro-2H-pyran (42.88 ml; 494 mmol) in toluene (60 ml) is added over 90 minutes. The mixture is further stirred at 50°C for 90 minutes. The resulting solution is washed twice with 0.5N aqueous sodium hydroxide solution (96 ml each) and twice with water (96 ml each). The resulting turbid organic phase is concentrated in vacuo using an impeller stirrer to afford a mixture of 5-(2-chlorophenyl)-2-(tetrahydropyran-2-yl)-2H-tetrazole (N2-isomer) and 5-(2-chlorophenyl)-1-(tetrahydropyran-2-yl)-1H-tetrazole (N1-isomer) in a ratio of about 95:5 (according to ¹H-NMR) as a yellow liquid.
¹H-NMR of N2-isomer (400 MHz, CDCl₃): 1.72-1.84 (m, 3 H), 2.16-2.25 (m, 2 H), 2.46-2.55 (m, 1 H), 3.80-3.86 (m, 1 H), 4.02-4.07 (m, 1 H), 6.12-6.14 (m, 1 H), 7.36-7.44 (m, 2 H), 7.52-7.56 (m, 1 H), 7.96-7.98 (m, 1 H).
¹H-NMR of N1-isomer (400 MHz, CDCl₃): 5.44-5.47 (m, 1 H). Characteristic signal which does not overlap with the signals of the N2-isomer.

### Example 9:

### 5-(2-Bromophenyl)-2-(tetrahydropyran-2-yl)-2H-tetrazole and 5-(2-Bromophenyl)-1-(tetrahydropyran-2-yl)-1 H-tetrazole.

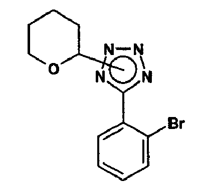

A suspension of 5-(2-bromophenyl)-1 H-tetrazole (4.50 g; 20.0 mmol) in tert-butyl methyl ether (40 ml) is warmed to 45°C and methanesulfonic acid (0.058 g; 0.60 mmol) is added. A solution of 3,4-dihydro-2H-pyran (1.90 ml; 21 mmol) in tert-butyl methyl ether (21 ml) is added to the resulting mixture over 1 hour at 45°C. The mixture is further stirred for 6 hours at 45°C. The resulting solution is cooled to about 0°C and a solution of sodium hydrogencarbonate (2.4 g) in water (30 ml) is added. The aqueous phase is separated and extracted with tert-butyl methyl ether (10 ml). The combined organic phases are washed twice with a 1 N KOH solution (10 ml each) and once with a solution of 10 weight-% of sodium chloride in water (10 ml). The resulting organic phase is dried over anhydrous sodium sulfate, filtered and evaporated in vacuo to afford a mixture of 5-(2-bromophenyl)-2-(tetrahydropyran-2-yl)-2H-tetrazole (N2-isomer) and 5-(2-bromophenyl)-1-(tetrahydropyran-2-yl)-1 H-tetrazole (N1-isomer) in a ratio of about 93:7 (according to ¹H-NMR) as an orange oil.
¹H-NMR of N2-isomer (400 MHz, CDCl₃): 1.72-1.85 (m, 3 H), 2.18-2.26 (m, 2 H), 2.45-2.54 (m, 1H), 3.80-3.86 (m, 1 H), 4.01-4.07 (m, 1 H), 6.12-6.15 (m, 1 H), 7.31-7.35 (m, 1 H), 7.41-7.45 (m, 1 H), 7.73-7.75 (m, 1 H), 7.87-7.90 (m, 1 H).

### Example 10:

### 5-(4'-Diethoxymethyl-biphenyl-2-yl)-2-(tetrahydropyran-2-yl)-2H-tetrazole.

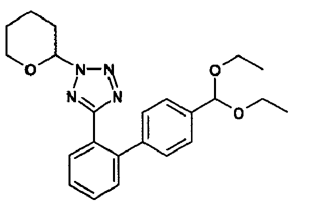

To a suspension of magnesium turnings (5.11 g) in anhydrous tetrahydrofuran (40 ml) is added 1,2-dibromoethane (0.106 ml; 1.2 mmol). The suspension is cooled to 12°C and 6 ml of a solution of 1-bromo-4-(diethoxymethyl)benzene (53.6 g; 200 mmol) in anhydrous tetrahydrofuran (120 ml) is added. After the reaction has started the remainder of the solution of 1-bromo-4-(diethoxymethyl)benzene is added over 90 minutes. The resulting mixture is further stirred at 20 to 25°C for 2.5 hours. The mixture is diluted with anhydrous tetrahydrofuran to a total volume of 250 ml affording a solution of the corresponding arylmagnesium bromide of about 0.78 M concentration. Under anhydrous conditions, 15.0 ml of the above 0.78 M arylmagnesium bromide solution (11.7 mmol) is cooled to about 0°C and a 0.5 M zinc chloride solution in tetrahydrofuran (23.4 ml; 11.7 mmol) is added over 15 minutes. The resulting suspension is stirred at room temperature for 30 minutes in order to complete the formation of the corresponding arylzinc reagent. In another flask, a solution of a mixture of 5-(2-bromophenyl)-2-(tetrahydropyran-2-yl)-2H-tetrazole and 5-(2-bromophenyl)-1-(tetrahydropyran-2-yl)-1H-tetrazole (2.78 g; 9.0 mmol) in tetrahydrofuran (9 ml) is added to dichlorobis(triphenylphosphine)palladium(II) (0.253 g; 0.36 mmol) under anhydrous conditions. To the vigorously stirred resulting yellow-orange suspension is added at room temperature the above suspension of the arylzinc reagent over 40 minutes. The mixture is further stirred at room temperature for 17 hours. A solution of sodium hydrogencarbonate (1.2 g) in water (15 ml) and ethyl acetate (20 ml) are then added. The aqueous phase is separated and extracted with ethyl acetate (60 ml). The combined organic phases are washed twice with a solution of sodium hydrogencarbonate (1.2 g) in water (15 ml each) and twice with water (15 ml each) and are evaporated in vacuo. The resulting yellow-orange oil is dissolved in a small volume of tert-butyl methyl ether, filtered over a filter aid, evaporated in vacuo and purified by column chromatography on silica gel eluting with a 1:4 mixture of ethyl acetate and hexane to afford the main isomer (N2-isomer) 5-(4'-diethoxymethyl-biphenyl-2-yl)-2-(tetrahydropyran-2-yl)-2H-tetrazole as a colorless oil.
¹H-NMR of N2-isomer (400 MHz, CDCl₃): 1.24 (t, *J* = 7.2 Hz, 6 H), 1.61-1.66 (m, 3 H), 1.88-2.03 (m, 2 H), 2.11-2.18 (m, 1 H), 3.50-3.71 (m, 6 H), 5.49 (s, 1 H), 5.97-5.99 (m, 1 H), 7.18-7.20 (m, 2 H), 7.38-7.40 (m, 2 H), 7.43-7.56 (m, 3 H), 7.90-7.92 (m, 1 H).
Mass spectrum (ES+): m/z = 409 [M+H]⁺

### Example 11:

### 2' -(2H-tetrazol-5-yl)biphenyl-4-carbaldehyd.

To 5-(4'-Diethoxymethyl-biphenyl-2-yl)-2-(tetrahydropyran-2-yl)-2H-tetrazole (0.408 g; 1.00 mmol) are added 94 % ethanol (2.5 ml) and a 2N aqueous solution of hydrochloric acid (0.5 ml; 1.0 mmol). The resulting solution is heated to 45°C for 3 hours. After the addition of water (about 2 ml) the mixture is allowed to cool down to room temperature and then stirred at 0 to 5°C for 30 minutes. The resulting suspension is filtered and the solids are washed with a small amount of water, dried in vacuo at 40°C to afford 2'-(1H-tetrazol-5-yl)-biphenyl-4-carbaldehyde as white, crystalline powder.
Melting point: 187.5-190.0°C.
High resolution mass spectrum (ES+): found: m/z = 251.0928 [M+H]⁺; calculated: m/z = 251.0927.

## Claims

1. A process for the manufacture of the compound of formula 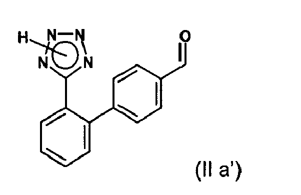 or a salt thereof, comprising
(i) reacting, in the presence of an acid, a compound of formula 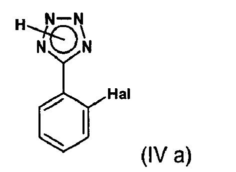 or a salt thereof, wherein Hal is halogen,
with a compound of formula 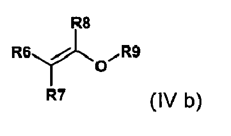 wherein
R₆, R₇ and R₈, independently of one another, are selected from hydrogen or C₁-C₆-alkyl
R₉ is C₁-C₆-alkyl
or R₇ and R₉ together form C₂-C₅-alkylene
or R₆ and R₈ together form C₃-C₆-alkylene; and
(ii) reacting, in the presence of a transition metal catalyst, a resulting compound of formula 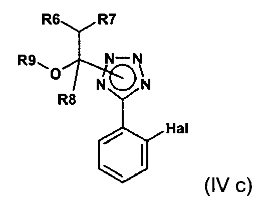 with a compound of formula 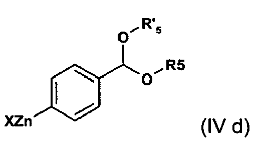 wherein
X is halogen;
R₅ and R'₅, independently of one another, are C₁-C₇-alkyl or together form C₂-C₄-alkylene; and
(iii) removing sequentially or in a single step the protecting groups from a resulting compound of formula 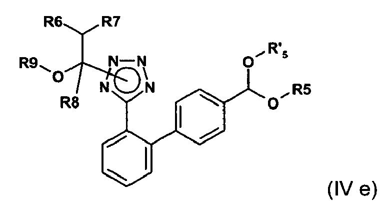 by treatment with an acid
(iv) isolating a resulting compound of formula (IIa') or a salt thereof.

2. The process according to claim 1 wherein step (i) is carried out in the presence of a Bronstedt acid.

3. The process according to claim 1 or 2 wherein step (ii) is carried out by using a platinium or palladium catalyst.

4. The process according to any one of claims 1 to 3 wherein step (iii) is carried out by dissolving the compound of formula (IV e) in water or a mixture of water and an appropriate organic solvent and subsequent treating with an acid at elevated temperature.

5. The process according to any one of claims 1 to 4 wherein the compound of formula (lVd) is prepared
i) by reacting a compound of formula 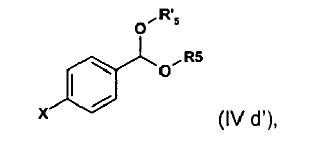 , wherein X is halogen,
with magnesium under Grignard conditions, to form a compound of formula 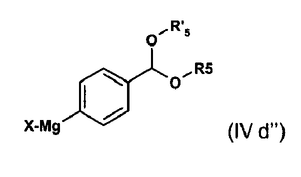
ii) by treating the reselting compound of formula (IVd") with Zn(X)₂
wherein X is halogen.

6. The process according to claim 5 wherein the the reaction of the compound of formula (lVd') with magnesium under Grignard conditions is executed in the presence of an activator

7. A compound of formula 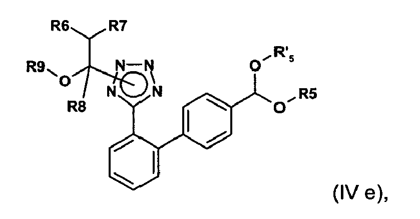 wherein
R₅ and R'₅, independently of one another, are C₁-C₇-alkyl, or together form C₂-C₄-alkylene;
R₆; R₇ and R₈, independently of one another, are hydrogen or C₁-C₇-alkyl or R₆ and R₈ together form C₃-C₆-alkylene;
R₉ is C₁-C₇-alkyl, or R₇ and R₉ together form C₂-C₅-alkylene.

8. The compound of claim 7 wherein
R₅ and R'₅, independently of one another, are C₁-C₄-alkyl, or together form C₂-C₄-alkylene;
R₆; R₇ and R₈, independently of one another, are hydrogen or C₁-C₄-alkyl; and
R₉ is C₁-C₄-alkyl or R₇ and R₉ together form C₂-C₅-alkylene.

9. The compound according to claim 7 or 8 wherein
R₅ and R'₅, independently of one another, are C₁-C₃-alkyl; or
R₆; R₇ and R₈, independently of one another, are hydrogen and R₉ is C₁-C₄-alkyl.

10. The compound according to any one of claims 7 to 9 wherein
R₅ and R'₅, independently of one another, are C₁-C₃-alkyl;
R₆ and R₈ are hydrogen; and
R₇ and R₉ together form C₂-C₃-alkylene.

11. The compound of claim 7 having the formula
